(19) Europäisches Patentamt

European Patent Office

Office européen des brevets

(11) **EP 0 520 334 B1**

(12) **EUROPÄISCHE PATENTSCHRIFT**

(45) Veröffentlichungstag und Bekanntmachung des Hinweises auf die Patenterteilung:
**24.01.1996 Patentblatt 1996/04**

(51) Int. Cl.$^6$: **C01B 39/00**, B01J 29/04, C10G 35/06, C07C 2/00, C07C 15/00

(21) Anmeldenummer: **92110381.8**

(22) Anmeldetag: **19.06.1992**

(54) **Kristallines, zeolithanaloges Gallosilicat und Verfahren zu dessen Herstellung**

Crystalline zeolite-like gallosilicate and process for its preparation

Gallosilicate cristallin analogue à une zéolite et son procédé de préparation

(84) Benannte Vertragsstaaten:
**BE DE ES FR GB IT NL**

(30) Priorität: **25.06.1991 DE 4120847**

(43) Veröffentlichungstag der Anmeldung:
**30.12.1992 Patentblatt 1992/53**

(73) Patentinhaber:
- **AlSi-PENTA Zeolithe GmbH**
  **D-92421 Schwandorf (DE)**
  Benannte Vertragsstaaten:
  **DE FR IT NL**
- **VEBA OEL AG**
  **D-45896 Gelsenkirchen (DE)**
  Benannte Vertragsstaaten:
  **BE DE ES FR GB IT NL**

(72) Erfinder:
- **Wallau, Martin Dr.**
  **W-6500 Mainz 42 (DE)**

- **Spichtinger, Rudolf**
  **W-6230 Frankfurt 80 (DE)**
- **Unger, Klaus Konradin Prof. Dr.**
  **W-6140 Bensheim 8 (DE)**
- **Tissler, Arno Dr.**
  **W-5300 Bonn 1 (DE)**
- **Thome, Roland Dr.**
  **W-5300 Bonn 1 (DE)**

(74) Vertreter: **Müller-Wolff, Thomas**
  **D-53704 Siegburg (DE)**

(56) Entgegenhaltungen:
  EP-A- 0 094 288      EP-A- 0 150 256
  EP-A- 0 323 893      EP-A- 0 327 189
  EP-A- 0 443 539      FR-A- 1 563 559
  US-A- 4 803 060

Bemerkungen:
  Die Akte enthält technische Angaben, die nach dem Eingang der Anmeldung eingereicht wurden und die nicht in dieser Patentschrift enthalten sind.

EP 0 520 334 B1

**Beschreibung**

Die Erfindung betrifft ein kristallines, zeolithanaloges Gallosilikat mit Pentasilstruktur sowie ein Verfahren zu seiner Herstellung durch hydrothermale Kristallisation aus einem Reaktionsansatz, der im rein anorganischen wäßrig-alkalischem Medium $SiO_2$ und $Ga_2O_3$ bzw. deren hydratisierte Derivate oder Alkalisate oder Gallate oder deren Salze enthält.

Aus EP-A-0 443 539 ist ein Verfahren zur Herstellung von Gallosilikaten bekannt, bei dem als Siliziumquelle ein Feinstaub mit 86 bis 99 Gew.-% $SiO_2$ verwendet wird. Der Feinstaub besteht aus 86 bis 98 % Silizium und weiteren Bestandteilen, wie Eisenoxid, Aluminiumoxid, Kaliumoxid, Magnesiumoxid sowie Siliziumcarbid, die in einer Menge von bis zu 14 % im Feinstaub vorliegen können. Durch die Zugabe dieser Fremdbestandteile wird es ermöglicht, daß bei dem bekannten Verfahren auf die Zugabe von organischen, strukturdirigierenden Substanzen (Templaten) verzichtet werden kann.

In FR-A-1 563 559 ist ein synthetisches, kristallines Gallosilikat vom Typ Z14 GS beschrieben, das eine Faujasith-Struktur aufweist. Bei dem Verfahren zur Herstellung des bekannten Gallosilikats wird eine Kristallisationstemperatur von unterhalb 110°C angewandt.

Nach EP-A-0 323 893 wird bei einem Verfahren zur Herstellung von Zeolith L eine Mischung von Kalium und anderen Alkali-Metallen verwendet und auf einen Kristallisationsbeschleuniger verzichtet. Dies gilt auch für die EP-A-0 094 288 bei der zusätzlich als Reaktionspartner noch Wasserstofffluorosilikat eingesetzt wird.

Aus EP-A-0 327 189 ist eine Synthesevariante bekannt, bei der strukturlenkende bzw. strukturstabilisierende organische Verbindungen eingesetzt werden. Diese haben eine Reihe von schwerwiegenden Nachteilen, die eine großtechnische und gleichzeitig umweltschonende Herstellung ausschließen. Die bekannten Synthesevarianten sind zudem leicht entzündlich und stark korrosiv, so daß auch aus diesem Grund eine dauerhafte Anwendung ausscheidet.

Aufgabe der vorliegenden Erfindung ist es, ein zeolithanaloges Gallosilikat mit Pentasilstruktur zu entwickeln, aus dem Katalysatoren mit hoher Stabilität und verbessertem katalytischen Langzeitverhalten hergestellt werden können und bei denen weder toxische noch korrosive Reagenzien eingesetzt werden. Dabei soll insbesondere die Aktivität der Katalysatoren bei zumindest gleichbleibender Selektivität verstärkt werden. Das neue Gallosilikat soll sich insbesondere zur Herstellung von Katalysatoren für die Umsetzung kurzkettiger Kohlenwasserstoffe eignen.

Diese Aufgabe wird erfindungsgemäß durch die in den Ansprüchen 1 und 5 enthaltenden Merkmale gelöst. talle mit breiter Teilchengrößenverteilung entstehen, die eine Verringerung der Stabilität bzw. der Standzeit, bezogen auf die Aufrechterhaltung der katalytischen Eigenschaften, zur Folge haben. Zudem hat die bei den bisher bekannten ZAG vorliegende inhomogene Verteilung der aktiven Zentren einen ungünstigen Einfluß auf die Selektivität und den Umsatz bei der katalytischen Reaktion.

Aufgabe der vorliegenden Erfindung ist es daher, ein neues kristallines, zeolithanaloges Gallosilicat mit verbesserten Eigenschaften zu entwickeln, aus dem Katalysatoren mit hoher Stabilität und verbessertem katalytischen Langzeitverhalten hergestellt werden können. Dabei soll insbesondere die Aktivität der Katalysatoren bei zumindest gleichbleibender Selektivität verstärkt werden. Das neue Gallosilicat soll sich insbesondere zur Herstellung von Katalysatoren für die Umsetzung kurzkettiger Kohlenwasserstoffe eignen. Eine weitere Aufgabe der vorliegenden Erfindung ist die Entwicklung eines umweltfreundlichen, templatfreien Verfahrens zur Herstellung eines derartigen kristallinen, zeolithanalogen Gallosilicates.

Diese Aufgabe wird erfindungsgemäß gelöst durch ein Gallosilicat mit den charakteristischen Merkmalen gemäß Anspruch 1 sowie das Verfahren mit den im Anspruch 5 genannten Merkmalen.

Das erfindungsgemäße Gallosilicat weist eine weitestgehend homogene Verteilung der Si- und Ga-Atome über den gesamten Kristall auf. Dabei ist der Wert des Atomverhältnisses Si/Ga an der äußeren Kristalloberfläche nicht größer als der durchschnittliche Wert dieses Verhältnisses im gesamten Kristall. Bedingt durch die homogene Verteilung der Ga-Atome sind die aktiven Zentren gleichmäßig über den gesamten Kristall verteilt. Die damit verbundene hohe Gesamtaktivität der aus dem erfindungsgemäßen Gallosilicat hergestellten Katalysatoren, führt zu einem gesteigerten Umsatz bei den katalytischen Reaktionen. Darüberhinaus ergibt sich durch die besondere Galliumverteilung eine erhöhte Langzeit-Stabilität der Katalysatoren, da die Degallilierung, d. h. das Herauslösen der Ga-Atome durch thermische Beanspruchung (= Deaktivierung) unter den Katalysebedingungen stark vermindert wird. Dieser Effekt wird durch die bei dem erfindungsgemäßen Gallosilicat vorliegende engere Teilchengrößenverteilung noch verstärkt. Ferner weisen die Katalysatoren aus dem neuen Gallosilicat eine höhere thermische und hydrothermale Belastbarkeit auf.

In einer bevorzugten Ausführungsform weist das erfindungsgemäße Gallosilicat ein oder mehrere Ionen aus der Gruppe $H^+$, $Na^+$ und $NH_4^+$ oder Kationen eines Metalls der Nebengruppe 8 des Periodensystems auf. Der entsprechende Ionenaustausch kann nach den hierbei üblichen Verfahren vorgenommen werden.

Der optimale Bereich für die Zusammensetzung des neuen Gallosilicates in dehydratisierter Form wird durch folgende Summenformel charakterisiert:

$$0,9 \pm 0,2 \ M_{2/n} \cdot Ga_2O_3 \cdot 5 - 1000 \ SiO_2$$

wobei M ein Kation und n dessen Wertigkeit ist. Dabei sollte das Gallosilicat zumindest die in Tabelle 1 angegebenen Netzebenenabstände aufweisen.

Tabelle 1

| Netzebenenabstände d der in den Beispielen 1 - 3 synthetisierten Gallosilikate | |
| --- | --- |
| d (Angström) | Intensität |
| $11,2 \pm 0,2$ | stark |
| $10,0 \pm 0,2$ | stark |
| $6,4 \pm 0,1$ | schwach |
| $5,95 \pm 0,1$ | schwach |
| $5,6 \pm 0,1$ | schwach |
| $3,87 \pm 0,05$ | stark |
| $3,83 \pm 0,05$ | stark |
| $3,76 \pm 0,05$ | schwach |
| $3,74 \pm 0,05$ | mittelstark |
| $3,66 \pm 0,05$ | schwach |
| $2,01 \pm 0,02$ | schwach |
| $1,99 \pm 0,02$ | schwach |

Es wurde gefunden, daß dieses erfindungsgemäße Gallosilicat auf einfache Weise aus rein anorganischen Reaktionsansätzen durch hydrothermale Kristallisation in einem Autoklaven hergestellt werden kann. Das für das erfindungsgemäße Verfahren verwendete Reaktionsgemisch besteht aus Wasser und Natronlauge sowie aus $SiO_2$ (amorph) und $Ga_2O_3$ bzw. deren hydratisierten Derivaten oder Alkalisilicaten, Alkaligallaten oder deren Salzen. Die molaren Verhältnisses in der Reaktionsmischung werden dabei wie folgt eingestellt:

$$SiO_2/Ga_2O_3 \geqq 5$$

$$OH^-/SiO_2 = 0,05 - 1,0$$

$$H_2O/SiO_2 = 10 - 1000.$$

Das erfindungsgemäße Verfahren verzichtet auf den Einsatz von Templaten und ist dementsprechend umweltfreundlich. Des weiteren wird dadurch die Entstehung von breiten Teilchengrößenverteilungen im Produkt vermieden. Da zudem keine Calcinierung zum Ausbrennen organischer Stoffe notwendig ist, wird das Risiko einer Beschädigung des Produktes vollständig eliminiert. Darüberhinaus wird ein Kristallines, zeolithanaloges Gallosilicat mit homogener Ga-Verteilung und allen damit verbundenen Vorteilen erzeugt.

Die Umsetzung der Reaktionsmischung erfolgt bevorzugt bei Temperaturen zwischen 40 und 300 °C und unter autogenem Druck. Unterhalb von 40 °C läuft die Umsetzungsreaktion unwirtschaftlich langsam ab, während oberhalb von 300 °C die Gefahr der Umwandlung des Produkt-Gallosilicates unter Bildung von Quarz und/oder Cristobalith besteht. In einer besonders bevorzugten Ausführungsform wird die hydrothermale Kristallisation im Temperaturbereich zwischen 150 und 225 °C unter autogenem Druck durchgeführt.

Nach dem erfindungsgemäßen Verfahren ist, wie bereits erwähnt, keine Calcinierung notwendig. Das erzeugte Gallosilicat kann daher unmittelbar nach der Kristallisation einem Ionenaustausch unterzogen werden.

Zur Beschleunigung der Kristallisationsreaktion hat es sich als günstig erwiesen, der Reaktionsmischung ein gealtertes, aber noch röntgenamorphes, Gallosilicat-Keimbildungsgel mit einem Atomverhältnis $Si/Ga \geqq 5$ zuzugeben. Auf diese Weise wird das gebildete Produkt-Gallosilicat stabilisiert und gleichzeitig die Entstehung unerwünschter Nebenphasen, wie z. B. Quarz und/oder Cristobalith unterdrückt. Zweckmäßigerweise wird ein Keimbildungsgel mit den folgenden molaren Verhältnissen verwendet:

$$SiO_2/Ga_2O_3 \geqq 5$$

$$OH^-/SiO_2 = 0,01 - 1,0$$

$$H_2O/SiO_2 = 10 - 1000.$$

Dabei hat es sich als besonders günstig erwiesen, ein Keimbildungsgel einzusetzen, dessen Molverhältnisse $H_2O/SiO_2$ und $OH^-/SiO_2$ kleiner oder gleich den entsprechenden Molverhältnissen im Reaktionsgemisch sind.

Das Keimbildungsgel wird bevorzugt einer Reaktionsmischung mit der folgenden molaren Zusammensetzung zugegeben:

$$SiO_2/Ga_2O_3 \geqq 5$$

$$OH^-/SiO_2 = 0,05 - 1,0$$

$$H_2O/SiO_2 = 20 - 100.$$

Grundsätzlich kann das gealterte Keimbildungsgel in beliebiger Menge in die Reaktionsmischung eingebracht werden. Aus verfahrensökonomischen Gründen sollte die Zugabemenge allerdings einen Wert von 50 Gew.-% des Gesamtansatzes nicht übersteigen. Zur Erzeugung eines geeigneten Keimbildungsgels wird die Alterung unter atmosphärischem Druck bei Temperaturen zwischen 0 und 100 °C in einem Zeitraum von 2 Stunden bis zu 100 Tagen durchgeführt. Zweck der Alterungsbehandlung ist die Bildung röntgenamorpher Keime, die eine schnelle Hochtemperatur-Bildung von ZAG im endgültigen Gel beschleunigen und stabilisieren. Zur Herstellung des Keimbildungsgels wird eine $SiO_2$-Quelle (vorzugsweise amorphe pyrogene Kieselsäure) mit einer NaOH-Lösung und einer sauren Lösung einer Ga-Verbindung (z. B. $GaCl_3$ in HCl) vermischt. Diese Mischung wird dann im angegebenen Temperaturbereich über einen entsprechenden Zeitraum gerührt.

Das erfindungsgemäße Gallosilicat hat sich aufgrund der bereits genannten Eigenschaften für die Herstellung von Katalysatoren und Adsorbentien als besonders geeignet erwiesen. Die vorteilhaften katalytischen Eigenschaften zeigen sich insbesondere bei der Aromatisierung niederer Kohlenwasserstoffe. Diese Umsetzungen erfolgen bei Temperaturen zwischen 300 und 800 °C, Drucken zwischen 0,1 und 100 bar und Katalysatorbelastungen von 0,1 bis 100 $h^{-1}$ WHSV (= Weight Hour Space Velocity). Bevorzugte Bereiche für derartige Anwendungen sind Temperaturen von 400 bis 600 °C, Drucke von 1 bis 10 bar und Katalysatorbelastungen von 1 bis 10 $h^{-1}$ WHSV.

Im folgenden wird die Erfindung anhand von Ausführungsbeispielen näher erläutert. Die Beispiele 1 bis 3 betreffen erfindungsgemäße Gallosilicate, die templatfrei hergestellt wurden. Im Beispiel 4 wurde zu Vergleichzwecken ein Gallosilicat mit Templat synthetisiert.

Die Elementverteilungen von Silizium und Gallium über den Kristallquerschnitt der Gallosilicate wurden mit einer Elektronenstrahl-Mikrosonde IEOL IXA-733 mit angeschlossenem DEC-Rechner PDP 11/23 bestimmt. Die Proben wurden in Harz eingebettet, mit Diamantpaste abgeschliffen und mit Gold besputtert. Die Elektronenstrahl-Messungen wurden mit einer Ausgangsspannung von 15 kV und 50 µA durchgeführt.

Die Messung der Proben aus den Beispielen 1 bis 3 wurde unter Auslenken des Elektronenstrahls durchgeführt, wobei Elementverteilung und Rasterelektronenaufnahmen des Kristalls auf Photopapier aufgezeichnet wurden (line profile).

Die Messung der Probe aus Beispiel 4 wurde unter schrittweiser Bewegung der Probe unter dem Elektronenstrahl bei einer Schrittweite von 1 µm durchgeführt (line scan). Die erhaltenen Werte wurden über Rechnei ausgewertet und die Elementverteilung über einen Plotter ausgegeben.

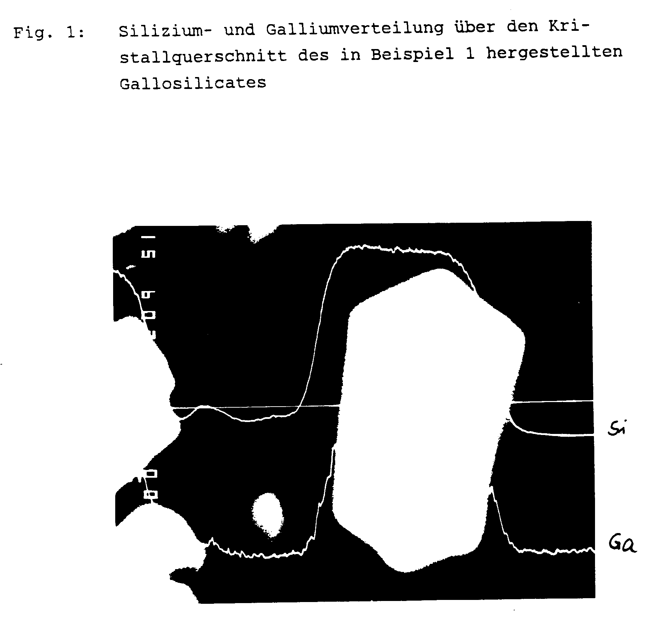

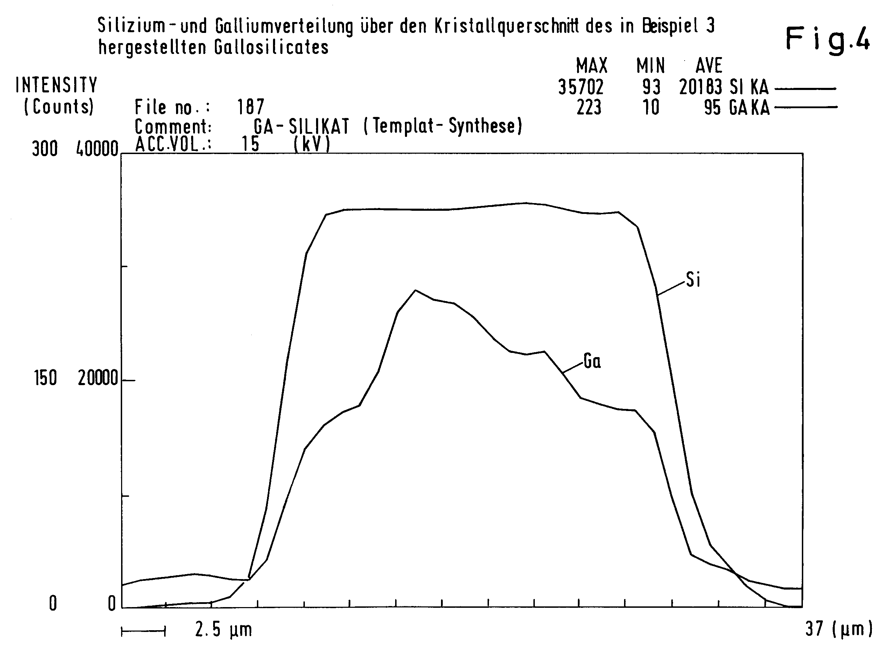

Wie die Elektronenstrahlmicroanalyse zeigt, weisen die erfindungsgemäßen ZAG (Beispiele 1 - 3) über den gesamten Kristall ein einheitliches Si-/Ga-Atomverhältnis auf (Fig. 1 - 3). Dabei ist der Wert des Atomverhältnisses Si/Ga der äußeren Kristalloberfläche nicht größer als der durchschnittliche Wert diese Verhältnisses im ganzen Kristall. Die mit Templat synthetisierte Probe (Beispiel 4), zeigt dagegen ein zur Kristallmitte hin deutlich abfallendes Si-/Ga-Atomverhältnis (Fig. 4).

In den Beispielen 5 bis 8 wurden die katalytischen Eigenschaften von Katalysatoren aus den Gallosilicaten der Beispiele 1 bis 4 anhand der Umsetzung von Propan zu BTX-Aromaten (Benzol, Toluol, Xylol) untersucht. Die Aromatisierung kurzkettiger Kohlenwasserstoffe erfolgt in der Regel mit den bei Erdölraffination und Steamcracken anfallenden $C_1$-$C_4$-Schnitten (W. Hölderich, Angew. Chemie Bd. 100, 232; 1988). Die bisherige Umwandlung dieser wertvollen Rohstoffe kann durch das hier beschriebene Upgrading-Verfahren deutlich verbessert werden. Als Edukt wird beispielsweise Propan eingesetzt, das mit hoher Selektivität in einer Dehydrocyclisierungsreaktion vornehmlich zu Benzol und Toluol umgewandelt wird. Man kann diese Stoffe zur Verbesserung der Klopffestigkeit von Ottokraftstoffen einsetzen (H. G. Franck, B. W. Stadelhofer, "Industrielle Aromatenchemie", Verlag Springer, Berlin 1987), oder sie als großtechnische Vorprodukte für die industrielle organische Chemie nutzen.

Die katalytischen Untersuchungen nach den Beispielen 5 bis 8 wurden in einem **Microreaktor,** der als Strömungsrohr arbeitet, durchgeführt. Der Katalysator befindet sich innerhalb einer Quarzschüttung, die von dem Reaktionsgas

($C_2$-$C_4$-Aliphaten, vorzugsweise Propan) durchströmt wird. Der Gasstrom wird über Nadelventile einreguliert. Die Beheizung des Reaktors erfolgt durch einen Rohrofen, der durch einen Eurothermregler mit Thyristorsteller gesteuert wird; das Thermoelement für die Steuerung des Temperaturreglers reicht in die Katalysatorschüttung. Die Katalysatoren werden bei der Reaktionstemperatur im Reaktor calciniert, nach ca. halbstündiger Stickstoffspülung leitet man das Reaktionsgas ein und analysiert das Reaktionsgemisch mit Hilfe eines on-line-geschalteten Gaschromatographen.

**Beispiel 1**

41,68 g amorphe pyrogene Kieselsäure wird mit 12,44 g NaOH und 2,59 g $GaCl_3$-Lösung (enthält 0,77 g Gallium) in 1000 g Wasser unter Rühren über 30 Minuten homogenisiert. Dieser Reaktionsansatz mit den molaren Verhältnissen $H_2O/SiO_2$=80, $SiO_2/Ga_2O_3$=129 und $OH^-/SiO_2$ = 0,42 wird in einen mit Teflon ausgekleideten Autoklaven mit einem Nenninhalt von 1,5 l gefüllt und bei 433 K 5 Tage unter autogenem Druck umgesetzt.

Nach Filtration und Waschung mit Wasser erhält man ca. 30 g eines kristallinen Gallosilikats mit einem molaren $SiO_2/Ga_2O_3$-Verhältnis von 95, das zumindest die in Tabelle 1 aufgeführten Netzebenenabstände und eine homogene Verteilung des Galliums über den Kristallquerschnitt aufweist (Fig. 1).

**Beispiel 2**

50,012 g amorphe pyrogene Kieselsäwe wird mit 13,317 g NaOH und 12,77 g $GaCl_3$-Lösung (enthält 1,935 g Ga) in 600 g Wasser unter Rühren über 30 Minuten homogenisiert. Diese Mischung mit den molaren Verhältnissen $H_2O/SiO_2$=40, $SiO_2/Ga_2O_3$=60, $OH^-/SiO_2$=0,3 wird 30 Tage bei 298 K gealtert, unter Rührung bei atmosphärischem Druck.

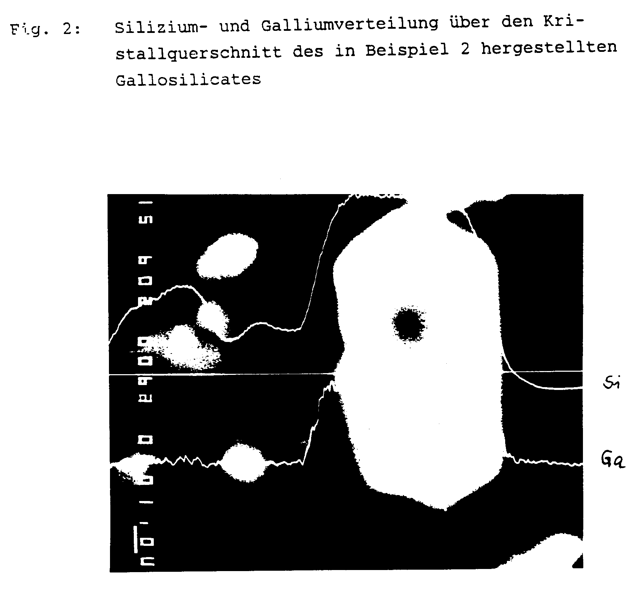

Anschließend wird eine zweite, gut homogenisierte Mischung aus 20,839 g amorphe pyrogene Kieselsäure, 9,092 g NaOH und 5,343 g $GaCl_3$-Lösung (enthält 0,809 g Gallium) in 1100 g Wasser hinzugefügt und der erhaltene Reaktionsansatz mit den molaren Verhältnissen $H_2O/SiO_2$=80, $SiO_2/Ga_2O_3$=60 und $OH^-/SiO_2$=0,375 in einen Edelstahlautoklaven mit einem Nennvolumen von 2 l überführt und bei 453 K unter Rühren 32 Stunden unter autogenem Druck umgesetzt. Nach Filtration und Waschung mit Wasser erhält man ca. 40 g eines kristallinen Gallosilikats mit einem molaren $SiO_2/Ga_2O_3$-Verhältnis von 42, das zumindest die in Tabelle 1 aufgeführten Netzebenenabstände und eine homogene Verteilung des Galliums über den Kristallquerschnitt aufweist (Fig. 2).

**Beispiel 3**

29,173 g amorphe pyrogene Kieselsäure wird mit 8,73 g NaOH und 11,178 g $GaCl_3$-Lösung (enthält 1,693 g Gallium) in 350 g Wasser unter Rühren über 30 Minuten homogenisiert. Diese Mischung mit den molaren Verhältnissen $H_2O/SiO_2$=40, $SiO_2/Ga_2O_3$=40, $OH^-/SiO_2$=0,3 wird 7 Tage bei 363 K unter atmosphärischem Druck gealtert. Anschließend wird eine gut homogenisierte Mischung aus 12,501 g amorpher pyrogener Kieselsäure, 5,826 g NaOH und 4,781 g $GaCl_3$-Lösung (enthält 0,724 g Gallium) in 350 g Wasser hinzugefügt und der erhaltene Reaktionsansatz mit den molaren Verhältnissen $H_2O/SiO_2$=80, $SiO_2/Ga_2O_3$=40 und $OH^-/SiO_2$=0,375 in einen Edelstahlautoklaven mit einem Nennvolumen von 1 l überführt und bei 453 K unter Rühren 21 Stunden unter autogenem Druck umgesetzt.

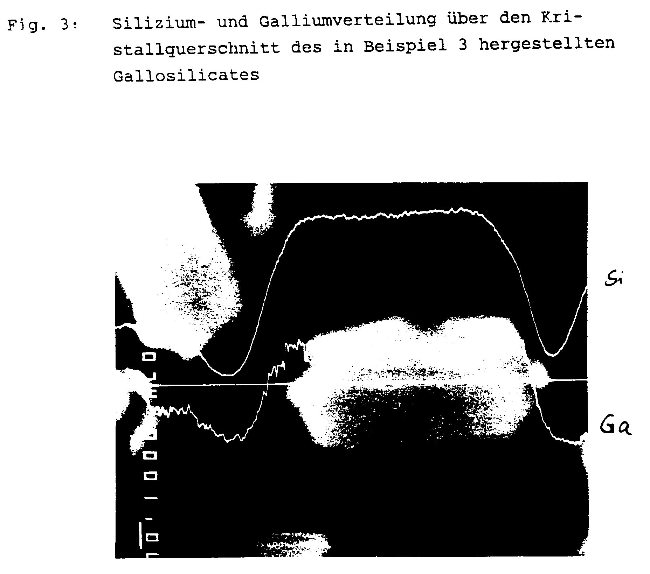

Nach Filtration und Waschung mit Wasser erhält man ca. 25 g eines kristallinen Gallosilikats mit einem molaren $SiO_2/Ga_2O_3$-Verhältnis von 26, das zumindest die in Tabelle 1 aufgeführten Netzebenenabstände und eine homogene Verteilung des Galliums über den Kristallquerschnitt aufweist (Fig. 3).

**Beispiel 4 (Vergleichsbeispiel)**

6,615 g kolloidales Silica-Sol (enthält 2,778 g $SiO_2$) wird mit 1,723 g Tetrapropylammoniumbromid (TPABr)), 0,445 g $GaCl_3$-Lösung (enthält 0,067 g Gallium) und 3,238 g Hexamethylentetramin (HMT) in 25 g Wasser unter Rühren 90 Minuten homogenisiert.

Dieser Reaktionsansatz mit den molaren Verhältnissen $H_2O/SiO_2$ = 30, $SiO_2/Ga_2O_3$ = 92, $HMT/SiO_2$ = 0,5, $TPABr/SiO_2$ = 0,14 wird in einen mit Teflon ausgekleideten Autoklaven mit einem Nenninhalt von 50 ml gefüllt und bei 453 K vier Tage unter autogenem Druck umgesetzt. Nach Filtration und Waschung mit Wasser erhält man ca. 2 g eines kristallinen alkalifreien Gallosilikates mit einem molaren $SiO_2/Ga_2O_3$-Verhältnis von 92, das zumindest die in Tabelle 1 aufgeführten Netzebenenabstände und eine ungleichmäßige Verteilung des Galliums (starke Anreicherung im Kristallinnern) über den Kristallquerschnitt aufweist (Fig. 4).

**Beispiel 5**

Das in Beispiel 1 hergestellte Gallosilikat wird durch dreimaligen Ionenaustausch mit 1 molarer Ammoniumnitrat-Lösung bei 95 °C über je 2 Stunden, wobei jeweils nach Beendigung eines Ionentauschvorganges mit destilliertem Wasser gewaschen wird, in die Ammoniumform überführt.
Nach Trocknung bei 120 °C über 12 h wird das Gallosilikat mit 30 Gew.-% einer amorphen Kieselsäure nach dem in EP 0 403 966 A1 beschriebenen Verfahren zu Extrudaten geformt, bei 550 °C über 12 Stunden im Reaktor im Stickstoffstrom aktiviert. Der Katalysator zeigt bei der Reaktionstemperatur von 530 °C und einer WHSV von 1 $h^{-1}$ (gr. Propan/gr. Katalysator · h) einen Propanumsatz von 15,3 % bei einer Selektivität zu BTX-Aromaten von 42 % (Tabelle 2).

**Beispiel 6**

Das in Beispiel 2 hergestellte Gallosilikat wird durch dreimaligen Ionenaustausch mit 1 molarer Ammoniumnitrat-Lösung bei 95 °C über je 2 Stunden, wobei jeweils nach Beendigung eines Ionentauschvorganges mit destilliertem Wasser gewaschen wird, in die Ammoniumform überführt.
Nach Trocknung bei 120 °C über 12 h wird das Gallosilikat mit 30 Gew.-% einer amorphen Kieselsäure nach dem in EP 0 403 966 A1 beschriebenen Verfahren zu Extrudaten geformt, bei 550 °C über 12 Stunden im Reaktor im Stickstoffstrom aktiviert. Der Katalysator zeigt bei der Reaktionstemperatur von 530 °C und einer WHSV von 1 $h^{-1}$ (gr. Propan/gr. Katalysator · h) einen Propanumsatz von 24,1 % bei einer Selektivität zu BTX-Aromaten von 50 % (Tabelle 2).

**Beispiel 7**

Das in Beispiel 3 hergestellte Gallosilikat wird durch dreimaligen Ionenaustausch mit 1 molarer Ammoniumnitrat-Lösung bei 95 °C über je 2 Stunden, wobei jeweils nach Beendigung eines Ionentauschvorganges mit destilliertem Wasser gewaschen wird, in die Ammoniumform überführt.
Nach Trocknung bei 120 °C über 12 h wird das Gallosilikat mit 30 Gew.-% einer amorphen Kieselsäure nach dem in EP 0 403 966 A1 beschriebenen Verfahren zu Extrudaten geformt, bei 550 °C über 12 Stunden im Reaktor im Stickstoffstrom aktiviert. Der Katalysator zeigt bei der Reaktionstemperatur von 530 °C und einer WHSV von 1 $h^{-1}$ (gr. Propan/gr. Katalysator · h) einen Propanumsatz von 40,5 % bei einer Selektivität zu BTX-Aromaten von 51 % (Tabelle 2).

**Beispiel 8 (Vergleichsbeispiel)**

Das in Beispiel 4 hergestellt Gallosilikat wird durch Kalzination über 12 h bei 550 °C in die katalytisch aktive H-Form überführt und anschließend mit
30 Gew.-% einer amorphen Kieselsäure nach dem in EP 0 403 966 A1 beschriebenen Verfahren zu Extrudaten geformt, bei 550 °C über 12 Stunden im Reaktor im Stickstoffstrom aktiviert. Der Katalysator zeigt bei der Reaktionstemperatur von 530 °C und einer WHSV von 1 $h^{-1}$ (gr. Propan/gr. Katalysator · h) einen Propanumsatz von 2,8 % bei einer Selektivität zu BTX-Aromaten von 40 % (Tabelle 2).

Tabelle 2

| Propanaromatisierungseigenschaften erfindungsgemäßer Katalysatoren (Beispiele 5 - 7) und eines Vergleichs-Katalysators (Beispiel 8). | | | |
|---|---|---|---|
| Beispiel Nr. | $SiO_2/Ga_2O_3$ [mol/mol] | Umsatz [ % ] | Selektiv zu BTX-Aromaten [ % ] |
| 5 | 95 | 15,3 | 42 |
| 6 | 42 | 24,1 | 50 |
| 7 | 26 | 40,5 | 51 |
| 8 | 92 | 2,8 | 40 |

Die Ergebnisse der Untersuchungen zum Vergleich von erfindungsgemäßen ZAG, die eine einheitliche Galliumverteilung aufweisen und ZAG mit uneinheitlicher Metallverteilung lassen sich wie folgt zusammenfassen:

1) Aliphaten sowie Cycloaliphaten (verzweigt und unverzweigt, gesättigt oder ungesättigt) mit einer Kohlenstoffzahl von zwei bis sechs können an allen untersuchten ZAG aromatisiert werden
(400 °C < T < 600; 0,1 bar < p < 100 bar; 0,1 $h^{-1}$ < WHSV < 100 $h^{-1}$).

2) ZAG mit höheren Galliumanteilen weisen, bei gleichen Reaktionsbedingungen (Temperatur, WHSV, Druck) höhere Umsätze und ähnliche Selektivitäten bezüglich der Bildung von BTX-Aromaten aus als solche mit geringeren Ga-Anteilen.

3) Katalysatoren aus den erfindungsgemäßen Gallosilicaten mit homogener Ga-Verteilung über den Kristallquerschnitt weisen deutlich höhere Umsätze bei zumindest gleichbleibender Selektivität bezüglich der Bildung von BTX-Aromaten auf als Katalysatoren aus Gallosilicaten mit einer Ga-Anreicherung im Kristallinneren.

**Patentansprüche**

1. Kristallines, zeolithanaloges Gallosilikat mit Pentasilstruktur,
dadurch gekennzeichnet,
daß der Wert des Si-/Ga-Atomverhältnisses der äußeren Kristalloberfläche nicht größer ist als der durchschnittliche Wert dieses Verhältnisses im ganzen Kristall, wobei das Gallosilikat ein oder mehrere Ionen aus der Gruppe $H^+$, $Na^+$, $NH_4^+$ oder Kationen eines Metalls der Nebengruppe 8 in der Tafel des periodischen Systems enthält.

2. Kristallines, zeolithanaloges Gallosilicat nach einem der vorhergehenden Ansprüche, dadurch gekennzeichnet, daß es in dehydratisierter Form die folgende chemische Zusammensetzung aufweist:

$$0,9 \pm 0,2 \ M_{2/n} \cdot Ga_2O_3 \cdot 5 - 1000 \ SiO_2$$

wobei M ein Kation und n dessen Wertigkeit ist.

3. Kristallines, zeolithanaloges Gallosilicat nach einem der vorhergehenden Ansprüche, dadurch gekennzeichnet, daß es zumindest die in Tabelle 1 aufgeführten Netzebenenabstände aufweist.

4. Verfahren zur Herstellung eines kristallinen, zeolithanalogen Gallosilikates mit Pentasilstruktur nach einem der vorhergehenden Ansprüche durch hydrothermale Kristallisation aus einem Reaktionsansatz, der im rein anorganischen wäßrig-alkalischen Medium $SiO_2$ und $Ga_2O_3$ beziehungsweise deren hydratisierte Derivate oder Alkalisilikate oder - Gallate oder deren Salze enthält,
dadurch gekennezeichnet,
daß dem Reaktionsansatz als Kristallisationsbeschleuniger ein gealtertes, aber noch röntgenamorphes Gallosilikatkeimbildungsgel mit einem Si/Ga-Atomverhältnis $\geq 5$ zugesetzt wird und
daß das Reaktionsgemisch die folgende chemische Zusammensetzung, ausgedrückt in Form der Molverhältnisse, aufweist:
$SiO_2/Ga_2O_3 \geq 5, OH^-/SiO_2 = 0,05 - 1,0, H_2O/SiO_2 = 10 - 1000$ und daß die hydrothermale Kristallisation im Temperaturbereich zwischen 150 und 225°C unter autogenem Druck durchgeführt wird.

5. Verfahren nach einem der vorhergehenden Ansprüche, dadurch gekennzeichnet, daß ein gealtertes Keimbildungsgel folgender chemischer Zusammensetzung, ausgedrückt in Form der Molverhältnisse, eingesetzt wird:

$$SiO_2/Ga_2O_3 \geq 5, \ OH^-/SiO_2 = 0,01 - 1,0, \ H_2O/SiO_2 = 10 - 1000.$$

6. Verfahren nach einem der vorhergehenden Ansprüche, dadurch gekennzeichnet, daß die Molverhältnisse $H_2O/SiO_2$ und $OH^-/SiO_2$ im gealterten Keimbildungsgel kleiner oder gleich den entsprechenden Molverhältnissen im Reaktionsgemisch sind.

7. Verfahren nach einem der vorhergehenden Ansprüche, dadurch gekennzeichnet, daß das gealterte Keimbildungsgel Reaktionsansätzen mit folgender molarer Zusammensetzung beigemischt wird:

$$SiO_2/Ga_2O_3 \geq 5, \ OH^-/SiO_2 = 0,05 - 1, \ H_2O/SiO_2 = 20 - 100.$$

8. Verfahren nach einem der vorhergehenden Ansprüche, dadurch gekennzeichnet, daß das gealterte Keimbildungsgel in Mengen von 0 bis 50 Gewichtsprozent, bezogen auf den Gesamtansatz, zugegeben wird.

9. Verfahren nach einem der vorhergehenden Ansprüche, dadurch gekennzeichnet, daß das Keimbildungsgel bei atmosphärischem Druck im Bereich von 0 bis 100 °C über zwei Stunden bis einhundert Tage gealtert wird.

**10.** Verfahren nach einem der vorhergehenden Ansprüche, dadurch gekennzeichnet, daß der Reaktionsansatz bei Temperaturen zwischen 40 und 300 °C unter autogenem Druck zu einem kristallinen, zeolithanalogen Gallosilicat umgesetzt wird.

**11.** Verfahren nach einem der vorhergehenden Ansprüche, dadurch gekennzeichnet, daß das kristalline, zeolithanaloge Gallosilicat direkt nach der Kristallisation einem Ionentausch unterzogen wird.

**12.** Verwendung eines kristallinen, zeolithanalogen Gallosilicates nach einem der vorhergehenden Ansprüche zur Herstellung von Katalysatoren oder Adsorbentien.

**13.** Verwendung nach Anspruch 15 zur Herstellung eines Katalysators für die Umsetzung von Kohlenwasserstoffverbindungen.

**14.** Verwendung nach Anspruch 16 zur Herstellung eines Katalysators für die Aromatisierung niederer Kohlenwasserstoffe im Bereich von 300 bis 800 °C, vorzugsweise 400 bis 600 °C, im Druckbereich von 0,1 - 100 bar, vorzugsweise zwischen 1 und 10 bar und Katalysatorbelastungen zwischen 0,1 und 100 $h^{-1}$ WHSV, vorzugsweise 1 - 10 $h^{-1}$ WHSV.

## Claims

1.  A crystalline, zeolite-analogous gallosilicate with a pentasil structure,
    characterised in
    that the value of the Si/Ga atom ratio of the outer crystal surface is no greater than the average value of said ratio in the entire cyrstal, with the gallosilicate containing one or several ions of the group of $H^+$, $Na^+$, $NH_4^+$ or cat ions of a metal of the subgroup 8 in the table of the periodic system.

2.  A crystalline, zeolite-analogous gallosilicate according to claim 1,
    characterised in
    that, in the dehydrated form, it comprises the following chemical composition:

    $$0.9 \pm 0.2 \, M_{2/n} \, x \, Ga_2O_3 \, x \, 5 - 1000 \, SiO_2,$$

    with M being a cation and n the value thereof.

3.  A crystalline, zeolite-analogous gallosilicate according to any one of the preceding claims,
    characterised in
    that it comprises at least the lattice distances as listed in Table 1.

4.  A process of producing a crystalline, zeolite-analogous gallosilicate with a pentasil structure according to any one of the preceding claims by hydrothermal crystallisation from a reaction mixture which, in the purely inorganic aqueous-alkaline medium, contains $SiO_2$ and $Ga_2O_3$ and, respectively, its hydratised derivatives or alkali silicates or gallates or their salts,
    characterised in
    that a crystallisation accelerating agent in the form of an aged, but still X-amorphous gallosilicate nucleation gel with a Si/Ga atom ratio $\geq 5$ is added to the reaction mixture and that the reaction mixture comprises the following chemical composition expressed in the form of the molar ratios: $SiO_2/Ga_2O_3 \geq 5$, $OH^-/SiO_2 = 0.05 - 1.0$, $H_2O/SiO_2$ = 10 - 1000 and that hydrothermal crystallisation is carried out in the temperature range between 150 and 225 °C under autogenous pressure.

5.  A process according to any one of the preceding claims, characterised in
    that an aged nucleation gel of the following chemical composition expressed in the form of the molar ratios is used:

    $$SiO_2/Ga_2O_3 \geq 5, \ OH^-SiO_2 = 0.01 - 1.0, \ H_2O/SiO_2 = 10 - 1000.$$

6.  A process according to any one of the preceding claims, characterised in
    that the molar ratios $H_2O/SiO_2$ and $OH^-/SiO_2$ in the aged nucleation gel are smaller than or equal to the corresponding molar ratios in the reaction mixture.

7. A process according to any one of the preceding claims, characterised in
that the aged nucleation gel is added to reaction mixtures with the following molar composition:

$$SiO_2/Ga_2O_3 \geqq 5, OH^-/SiO_2 = 0.05 - 1, H_2O/SiO_2 = 20 - 100.$$

8. A process according to any one of the preceding claims,
characterised in
that the aged nucleation gel is added in quantities of 0 to 50 percent by weight, with reference to the total mixture.

9. A process according to any one of the preceding claims,
characterised in
that the nucleation gel is aged at atmospheric pressure in the range of 0 to 100 °C over two hours to one hundred days.

10. A process according to any one of the preceding claims,
characterised in
that the reaction mixture is converted at temperatures ranging between 40 and 300 °C at autogenous pressure into a crystalline, zeolite-analogous gallosilicate.

11. A process according to any one of the preceding claims,
characterised in
that immediately after crystallisation, the crystalline, zeolite-analogous gallosilicate is subjected to an ion exchange.

12. Using a crystalline, zeolite-analogous gallosilicate according to any one of the preceding claims for producing catalysts or adsorbents.

13. Use according to claim 12 for producing a catalyst for converting hydrocarbon compounds.

14. Use according to claim 13 for producing a catalyst for the aromatisation of low hydrocarbons in the region of 300 to 800 °C, preferably 400 to 600 °C, in the pressure range of 0.1 - 100 bar, preferably between 1 and 10 bar, with catalyst loads between 0.1 and 100 $h^{-1}$ WHSV, preferably 1 - 10 $h^-_1$ WHSV.

**Revendications**

1. Gallosilicate cristallin, analogue à de la zéolite ayant une structure de pentasil, caractérisé en ce que la valeur du rapport atomique Si/Ga de la surface cristalline extérieure n'est pas plus grande que la valeur moyenne de ce rapport dans l'ensemble du cristal, le gallosilicate contenant un ou plusieurs ions choisis parmi $H^+$, $Na^+$, $NH_4^+$ ou des cations d'un métal du sous-groupe 8 du Tableau du Système Périodique (des éléments).

2. Gallosilicate cristallin, analogue à de la zéolite selon la revendication précédente, caractérisé en ce que, sous forme déshydratée, il présente la composition chimique suivante :

$$0,9 \pm 0,2\ M_2/n\ .\ Ga_2O_3\ .\ 5 - 1000\ SiO_2,$$

dans laquelle M représente un cation et n est sa valence.

3. Gallosilicate cristallin, analogue à de la zéolite, selon une des revendications précédentes, caractérisé en ce qu'il présente au moins les distances entre plans réticulaires indiquées au tableau 1.

4. Procédé de préparation d'un gallosilicate cristallin, analogue à de la zéolite, ayant une structure de pentasil, selon l'une des revendications précédentes, par cristallisation hydrothermale à partir d'une charge de réaction contenant, dans un milieu minéral hydro-alcalin pur $SiO_2$ et $Ga_2O_3$ ou leurs dérivés d'hydratation ou des silicates alcalins ou des gallates alcalins ou leurs sels,
procédé caractérisé en ce que l'on ajoute à la charge de réaction, à titre d'accélérateur de la cristallisation, un gel de gallosilicate vieilli mais encore amorphe aux rayons X et pouvant servir de gel formateur de germes, ayant un rapport atomique Si/Ga $\geqq$ 5 , et
en ce que le mélange réactionnel présente la composition chimique suivante, exprimée sous forme des rapports molaires:

$SiO_2/Ga_2O_3 \geqq 5$, $OH^-/SiO_2 = 0,05 - 1,0$ $H_2O/SiO_2 = 10 - 1000$, et en ce que la cristallisation hydrothermale est conduite dans un intervalle de la température compris entre 150 et 225°C sous la pression autogène.

5. Procédé selon la revendication précédente caractérisé en ce qu'on utilise un gel vieilli, formateur de germes et ayant la composition chimique suivante, exprimée sous forme des rapports molaires :

$$SiO_2/Ga_2O_3 \geqq 5, \ OH^-/SiO_2 = 0,01 - 1,0, \ H_2O/SiO_2 = 10\text{-}1000$$

6. Procédé selon l'une des revendications 4 ou 5 précédentes, caractérisé en ce que les rapports molaires $H_2O/SiO_2$ et $OH^-/SiO_2$ sont, dans le gel vieilli générateur de germes, inférieurs ou égaux aux rapports molaires correspondants dans le mélange réactionnel.

7. Procédé selon l'une des revendications 4 à 6 précédentes, caractérisé en ce que le gel vieilli générateur de germes est incorporé par mélangeage à des charges de réaction ayant la composition molaire suivante :

$$SiO_2/Ga_2O_3 \geqq 5, \ OH^-/SiO_2 = 0,05 - 1, \ H_2O/SiO_2 = 20 - 100.$$

8. Procédé selon l'une des revendications 4 à 7, caractérisé en ce que le gel vieilli, formateur de germes, est ajouté en des quantités de 0 à 50 % en poids, par rapport à la charge totale.

9. Procédé selon l'une des revendications 4 à 8 précédentes, caractérisé en ce que le gel formateur de germes est soumis à vieillissement sous la pression atmosphérique dans un intervalle de la température allant de 0 à 100°C pendant une période de temps de deux heures à 100 jours.

10. Procédé selon l'une des revendications 4 à 9 précédentes, caractérisé en ce que la charge de réaction est mise à réagir à des températures comprises entre 40 et 300°C sous la pression autogène, pour donner un gallosilicate cristallin analogue à de la zéolite.

11. Procédé selon l'une des revendications 4 à 10 précédentes caractérisé en ce que le gallosilicate cristallin, analogue à de la zéolite, est soumis, directement après la cristallisation, à un échange d'ions.

12. Utilisation d'un gallosilicate cristallin, analogue à de la zéolite, selon la revendication 1, pour la préparation de catalyseurs ou d'agents d'adsorption.

13. Utilisation selon la revendication 12, pour la préparation d'un catalyseur destiné à la réaction de composés hydrocarbonés.

14. Utilisation selon la revendication 13 pour préparer un catalyseur pour l'aromatisation d'hydrocarbures inférieurs, dans l'intervalle de la température allant de 300 à 800°C, avantageusement entre 400 et 600°C, dans l'intervalle de la pression compris entre 0,1 et 100 bars, avantageusement entre 1 et 10 bars et à des charges appliquées au catalyseur comprises entre 0,1 et 100 $h^{-1}$ de vitesse spatiale horaire en poids (poids par poids par heure), avantageusement 1 à 10 $h^{-1}$ de vitesse spatiale horaire en poids.

Fig. 1:    Silizium- und Galliumverteilung über den Kristallquerschnitt des in Beispiel 1 hergestellten Gallosilicates

Fig. 2:   Silizium- und Galliumverteilung über den Kri-
          stallquerschnitt des in Beispiel 2 hergestellten
          Gallosilicates

Fig. 3: Silizium- und Galliumverteilung über den Kristallquerschnitt des in Beispiel 3 hergestellten Gallosilicates

Silizium-und Galliumverteilung über den Kristallquerschnitt des in Beispiel 3 hergestellten Gallosilicates

Fig.4